# EUROPEAN PATENT SPECIFICATION

(11) **EP 3 885 353 B1**
(45) Date of publication and mention of the grant of the patent: **28.10.2026**
(21) Application number: 20165418.3
(22) Date of filing: 24.03.2020
(51) Int. Cl.: C07K 14/38, C12N 15/82

(54) **A VIRAL MOLECULE FOR PROTECTING PLANTS AGAINST C. ACUTATUM AND C. GLOESPORIOIDES FUNGI**
EIN VIRALES MOLEKÜL ZUM SCHUTZ VON PFLANZEN GEGEN C. ACUTATUM- UND C. GLOEOSPORIOIDES-FUNGUS
UNE MOLECULE VIRALE POUR PROTEGER LES PLANTES CONTRE LES FUNGUS C. ACUTATUM ET C. GLOEOSPORIOIDES

(43) Date of publication of application: 29.09.2021
(73) Proprietor: Universidade de Évora, 7000-803 Évora (PT)
(72) Inventor: Materatski, Patrick, 7005-591 Évora (PT); Campos, Maria, 7005-593 Évora (PT); Varanda, Carla, 7005-591 Évora (PT); Félix, Maria, 7005-401 Évora (PT); Peixe, Augusto, 7000-757 Évora (PT); Basaloco Fonseca, Margarida, Estremoz (PT)
(74) Representative: Pereira da Cruz, Joao

(56) References cited:
- WO-A2-02/090384
- XIAOQING SHI ET AL: "Efficient production of antifungal proteins in plants using a new transient expression vector derived from tobacco mosaic virus", PLANT BIOTECHNOLOGY JOURNAL, vol. 17, no. 6, 6 June 2019 (2019-06-06), GB, pages 1069 - 1080, XP055731335, ISSN: 1467-7644, DOI: 10.1111/pbi.13038
- J.M.S. CARDOSO ET AL.: "First characterization of infectious cDNA clones of olive mild mosaic virus", PHYTOPATHOLOGIA MEDITERRANEA, vol. 51, no. 2, 2012, pages 259 - 265, XP002800369
- DATABASE EMBL [online] 2 December 2011 (2011-12-02), "Olive mild mosaic virus isolate GP clone pUFLOMMV-5, complete genome.", XP002800370, retrieved from EBI accession no. EMBL:HQ651834 Database accession no. HQ651834
- CARLA M. R. VARANDA ET AL: "Genetic Diversity of the Coat Protein of Olive Mild Mosaic Virus (OMMV) and Tobacco Necrosis Virus D (TNV-D) Isolates and Its Structural Implications", PLOS ONE, vol. 9, no. 10, 28 October 2014 (2014-10-28), pages e110941, XP055730622, DOI: 10.1371/journal.pone.0110941

## Description

### Technical domain of the invention

The present invention relates to a novel virus-based vector for conferring protection of plants against *Colletotrichum acutatum* and *Colletotrichum gloesporioides* complexes, the pathogens that can cause anthracnose in plants.

In another aspect, the invention relates to a method for controlling said fungi in plants by applying a phytosanitary composition comprising a virus-based vector, carrying an antimicrobial sequence that provides plant protection against *C. acutatum* and *C*. *gloesporioides.*

Therefore, the present invention is in the technical field of plant disease control, microbiology and biotechnology.

### Background of the invention

Anthracnose is a major olive disease, caused by fungi from species within *Colletotrichum acutatum* and *Colletotrichum gloesporioides* complexes. This disease has a large impact in Portugal and also in Spain, Italy and other Mediterranean countries as well as in Australia, and South Africa. It also affects other crops with economic value, such as apple, citrus, stone fruits and others, which places the disease as a worldwide concern.

The pathogen forms abundant orange conidial masses and causes necrosis in fruits, which become damaged and may drop prematurely or mummify and the oil produced has poor quality. All plant organs may be affected, leading to necrosis, defoliation and death. The disease may result in high or total losses, and not only in cultivars with known susceptibility to the disease, such as the widely cultivated 'Galega vulgar', 'Cobrancosa' and others, but also in less susceptible cultivars such as 'Arbequina' and 'Picual' vastly cultivated through Portugal and Spain, which have shown great losses when conditions are favourable.

There are some aspects of the olive anthracnose cycle that are continuously being revealed. Recent studies suggested that the pathogen can move inside the plant from some organs to others that were not directly infected (Materatski et al., 2018), meaning that the plant acts as a reservoir of the fungus.

So far, anthracnose control strategies rely on synthetic fungicides that are unspecific, inefficient, expensive and harmful to the environment such as the ones disclosed in ES2116191.

Products currently used to control the disease, based on copper or penetrating products are not effective to prevent the disease as they only prevent new infections. In addition, they are harmful to the environment, not specific and expensive.

Several attempts were made to develop compositions based on microorganisms and/or natural compounds to address this situation such as the ones described in CN109221164 (A), WO2019033527 (A1), CN104694430 (A) and CN107927010 (A).

These documents disclose compositions comprising a combination of microorganism species or strains and plant extracts.

In particular, CN108690817 (A) discloses an antifungal composition comprising a combination of *Bacillus licheniformis* TG119 and *Aspergilus terreus* for controlling several plant diseases such as anthracnose.

Other approaches include the transformation of plants to include genomic sequences providing resistance to anthracnose, such as the one described in WO2019014584 (A1).

Nevertheless, anthracnose is still a major problem for fruit-tree production, in particular for olive production revealing that the formerly described approaches are not effective.

Therefore, there is an urgency to find new strategies to control this disease.

The present invention proposes a method based on a virus peptide that overcome the disadvantages of the prior art by providing a low cost, effective and rapid and selectively strategy against plant anthracnose, in particular affecting olive-trees and olive production. A further advantage is the environmentally friend use of the resulting products that are free from toxic chemicals and does not require genetic modification of plants, especially in the case of low growing rate plants as trees.

### Summary of the invention

The present invention relates to a novel virus-based vector for conferring protection of plants against *Colletotrichum acutatum* and *Colletotrichum gloesporioides* complexes, the pathogens that can cause anthracnose in plants. In another aspect, the invention relates to a method for controlling said fungi in plants by applying a composition comprising a virus-based vector, carrying an antimicrobial sequence that provides plant protection against *C*. *acutatum* and *C*. *gloesporioides.*

Thus, in one aspect the present invention relates to a vector based on a plant virus, which is modified to carry an antimicrobial sequence in plants thereby conferring them protection against the fungus *Colletotrichum acutatum* and/or *Colletotrichum gloesporioides* according to claim 1.

The resulting vector is useful to be produce phytosanitary compositions that can be used for controlling fungal diseases in plants caused by *Colletotrichum acutatum* and/or *Colletotrichum gloesporioides,* according to claim 2.

This product is of great interest for plant protection strategies, in particular concerning olive crop, since, when introduced in olive plants protects them from *C*. *acutatum* and *C. gloesporioides* fungi that cause anthracnose in olive, a disease of major concern for olive producers all over the world being responsible for extremely significant economic losses in this crop and that lacks efficient means of control.

The possibility of producing products free from toxic chemicals is particularly important in food quality and safety, as there is an increasing tendency for consumers to demand healthier products that are more valued than products from conventional agriculture.

In another aspect, the present invention relates to a method of controlling anthracnose caused by the fungus *Colletotrichum acutatum* and/or *Colletotrichum gloesporioides* by providing an adequate amount of a product comprising a vector as described above, according to claim 5.

This strategy is a breakthrough in fungi control, it is not harmful to the environment or public health and contributes to the end of the use of the great amounts of fungicides currently used in this crop to control this disease.

Additionally, it does not require plant transformation, generating lower costs and effective and rapid results.

The method of the invention allows to intensify and improve the olive production, also allowing the differentiation and anticipation of the productions, increasing the competitiveness of the agri-food sector.

### General description of the invention

The present invention relates to a virus molecule for protecting plants against *C*. *acutatum* and *C*. *gloesporioides* fungi.

The virus vector herein provided is based on *Olive mild mosaic virus* (OMMV) being the infectious transcript of OMMV cDNA clone obtained from an OMMV modified to become non-fungal transmitted. This virus mutant is further modified to cause no disease symptoms in plants and hereinafter named **OMMV_noSnoT.**

An antimicrobial sequence obtained from *Aspergillus niger* is amplified through Polymerase Chain Reaction (PCR) for producing blunt-ended DNA products.

This sequence is then ligated with T4 DNA ligase into OMMV_noSnoT, previously digested with the restriction enzyme HPaI leaving blunt ends.

The resulting vector comprising OMMV_noSnoT and Anafp (**SEQ. ID. 1** and **SEQ. ID. 2**), two sequences specifically designed for the present invention, is hereinafter termed **Collefree vector.** Collefree vector is stable, it replicates and accumulates in *Olea europaea* plants, causing no disease symptoms and is able to express the anti-microbial peptide Anafp in amounts that allow conferring protection of plants against *C*. *acutatum* and *C. gloesporioides.*

### 1. Construction of a virus vector

The virus vector of the present invention **(Collefree vector)** is based on *Olive mild mosaic virus* (OMMV), a virus originally isolated from olive by Cardoso *et al.* as described in Cardoso, J. M. S., Félix, M. R., Clara, M. I. E. & Oliveira, S. (2005). The complete genome sequence of a new necrovirus isolated from Olea europaea L. Arch Virol 150, 815-823.

The infectious transcript of OMMV cDNA clone obtained from an OMMV type-strain (Accession number HQ651834.1) is then modified to become non-fungal transmitted, through a single mutation from adenine to thymine at nt 3200 resulting in a non-synonymous change from asparagine to tyrosine at position 189 of the coat protein (CP) as described by Varanda et *al.* in Varanda, C.M.R.; Félix, M.R.; Soares, C.M.; Oliveira, S.; Clara, M.I.E. (2011). Specific amino acids of Olive Mild Mosaic Virus coat protein are involved on transmission by Olpidium brassicae. Journal of General Virology, 92 (9): 2209-2213.

The OMMV_noSnoT mutant virus is further modified to cause no disease symptoms in plants, through 3-point mutations: cytosine to guanine at nt 3359 (proline to alanine at position 242 of CP aa sequence); guanine to cytosine at nt 3365 (alanine to proline at position 244 of CP aa sequence) and guanine to thymine at nt 3368 (alanine to serine at position 245 of CP aa sequence.

Mutations are introduced using the QuikChange Site-Directed Mutagenesis kit Stratagene, in accordance to the instruction manual.

Primers designed for this specific purpose with the **SEQ.ID 3** and **SEQ.ID.4** are used according to the primer design guidelines:
**Mut_OMMV_Fwd:**
   **SEQ. ID. 3:** 5' GCTGGGGACGGCGGAGCTGTGCCTTCCACAGCTGTGGGC 3' and
**Mut_OMMV_Rev:**
   **SEQ. ID. 4:** 5' GCCCACAGCTGTGGAAGGCACAGCTCCGCCGTCCCCAGC;
wherein the mutations are represented in underlined.

Mutation strand synthesis reaction was, for a final volume of 50 µL, as follows: 5 µL of 10x reaction buffer, 50 ng of plasmid DNA (OMMV in puC18), 125 ng of **SEQ. ID. 3** and 125 ng of **SEQ. ID. 4,** 1 µL of dNTP mix and 2,5 U of PfuTurbo DNA polymerase.

Cycling parameters consisted of: 1 cycle at 95 °C for 30 seconds; 18 cycles at 95°C for seconds, 55 °C for 1 minute and 68 °C for 6 minutes.

Reaction was placed on ice for 2 minutes to cool the reaction. Digestion of amplification products was performed through Dpn I digestion to digest the parental dsDNA. 1 µL Dpn I restriction enzyme was added to the reaction and incubated at 37 °C for 1 hour.

Transformation was performed using XL1-Blue Supercompetent cells following the manufacturer's instructions.

An antimicrobial sequence obtained from *Aspergillus niger* (Accession number NT_166523.1, nucleotide 244873 to 245607) 735 nt in length, corresponding to the S-carboxyamidomethylated Anafp peptide (Lee et al., 1999) is amplified through Polymerase Chain Reaction PCR) using the following primers also specifically designed for the implementation of the present invention: **Anafp5'** (5'TCTTAACTACTCTACACCAC 3'), hereinafter **SEQ. ID. 1,** and **Anafp3'** (5' TTAAATAATAACAGCAAGTC 3') hereinafter **SEQ. ID. 2,** and the KOD Hot Start DNA Polymerase that inhibits the 3' - 5' exonuclease activity, producing blunt-ended DNA products.

Reaction consisted of 50 µL of: 1X buffer for KOD Start DNA Polymerase, 1,5 mM of MgSO₄, 0,2 mM of each dNTP, 0,3 µM of primer with SEQ.ID 1 (Anafp5'), 0,3 µL of primer with SEQ.ID.2 (Anafp3'), 1U KOD Hot Start DNA Polymerase and 10 ng of plasmid DNA of *A*. *niger.*

Cycling conditions consisted of one cycle at 95 °C for 2 min; 30 cycles of 95 °C for 20 seconds, 58 °C for 10 seconds and 70 °C for 1 minute.

The resulting sequence is then ligated with T4 DNA ligase into **OMMV_noSnoT,** previously digested with the restriction enzyme HPaI, that cuts in the position nt3454, 9 nt downstream of the CP and leaves blunt ends.

The resulting vector comprising OMMV_noSnoT plus Anafp, is hereinafter named **Collefree vector.** Collefree vector is stable, it replicates and accumulates in *Olea europaea* plants, causing no disease symptoms and is able to express the anti-microbial peptide Anafp in amounts that allow conferring protection of plants against *C*. *acutatum* and *C*. *gloesporioides.*

In short, it is obtained a virus-based vector (Collefree vector) for plant protection against C. acutatum and/or C. gloesporioides comprising the modified genome of Olive mild mosaic virus (OMMV) having the accession number HQ651834.1 with an antimicrobial sequence from Aspergillus niger having the accession number NT_166523.1, nucleotide 244873 to 245607, which is placed downstream of the CP the OMMV full genome with the following characteristics:
- A mutation from adenine to thymine at nt 3200;
- A mutation from cytosine to guanine at nt 3359;
- A mutation from guanine to cytosine at nt 3365;
- A mutation from guanine to thymine at nt 3368;
- A fragment of *A*. *niger* amplified by Anafp sequences **SEQ.ID.1** and **SEQ.ID.2,** the fragment being introduced at nt 3454.

### 2. Phytosanitary compositions comprising Collefree vector

Phytosanitary compositions for conferring protection of plants against anthracnose caused by *Colletotrichum acutatum* and *Colletotrichum gloesporioides* fungi can be produced comprising a virus-based vector as described in the previous section.

Said compositions can be presented in the form of suspension and spray that are directly applicable to plants to be treated.

### 3. Method for controlling anthracnose in plants

The virus-based vector described in the previous section, for conferring protection of plants against anthracnose caused by *Colletotrichum acutatum* and *Colletotrichum gloesporioides* fungi can be produced and maintained in Nicotiana benthamiana plants. Macerated leaves will be subjected to viral purification as described in Varanda et al., 2011. Viral suspensions will be used to spray inoculate plants, after loading the mixture into an airbrush attached to a compressor set which will be used to spray plant leaves.

### SEQUENCE LISTING

### (i) DNA sequence listing:

**SEQ.ID.1:** TCTTAACTACTCTACACCAC
**SEQ.ID.2:** TTAAATAATAACAGCAAGTC
**SEQ.ID.3:** GCTGGGGACGGCGGAGCTGTGCCTTCCACAGCTGTGGGC
**SEQ.ID.4:** GCCCACAGCTGTGGAAGGCACAGCTCCGCCGTCCCCAGC

## Claims

1. A **virus-based vector** (**Collefree vector**) for plant protection against *Colletotrichum acutatum* and/or *Colletotrichum gloesporioides* comprising on the modified genome of *Olive mild mosaic virus* (OMMV) having the accession number HQ651834.1 with an antimicrobial sequence from *Aspergillus niger* having the accession number NT_166523.1, nucleotide 244873 to 245607, which is placed downstream of the CP the OMMV full genome with:
- A mutation from adenine to thymine at nt 3200;
- A mutation from cytosine to guanine at nt 3359;
- A mutation from guanine to cytosine at nt 3365;
- A mutation from guanine to thymine at nt 3368;
- A fragment of *A*. *niger* amplified by Anafp sequences **SEQ.ID.1** and **SEQ.ID.2,** the fragment being introduced at nt 3454.

2. A **phytosanitary composition** for plant protection against *C. acutatum* and/or *C*. *gloesporioides* comprising a virus-based vector (**Collefree vector**) as described in claim 1.

3. A phytosanitary composition according to claim 2 comprising a suspension of the virus-based vector as described in claim 1.

4. A phytosanitary composition according to any of the claims 2 or 3 comprising in the form of spray to inoculate plants, after loading the mixture into an airbrush attached to a compressor set which is sprayed to the plant leaves.

5. A **method** for controlling anthracnose in plants comprising the administration of an adequate amount of a phytosanitary composition as described in any of the claims 2 to 4.

## Patentansprüche

1. **Virusbasierter Vektor** (**Collefree-Vektor**) zum Schutz von Pflanzen gegen *Colletotrichum acutatum* und/oder *Colletotrichum gloesporioides*, der das modifizierte Genom des *Olive mild mosaic virus* (OMMV) mit der Zugangsnummer HQ651834.1 sowie eine antimikrobielle Sequenz aus *Aspergillus niger* mit der Zugangsnummer NT_166523.1 umfasst, Nukleotide 244873 bis 245607, die stromabwärts des CP des vollständigen OMMV-Genoms angeordnet ist, mit:
- einer Mutation von Adenin zu Thymin bei Nt 3200;
- einer Mutation von Cytosin zu Guanin bei Nt 3359;
- einer Mutation von Guanin zu Cytosin bei Nt 3365;
- einer Mutation von Guanin zu Thymin bei Nt 3368;
- einem Fragment von *A. niger*, das durch die Anafp-Sequenzen **SEQ.ID.1** und **SEQ.ID.2** amplifiziert wurde, wobei das Fragment bei Nt 3454 eingefügt wurde.

2. **Phytosanitäre Zusammensetzung** zum Schutz von Pflanzen gegen *C. acutatum* und/oder *C. gloesporioides,* umfassend einen virusbasierten Vektor (**Collefree-Vektor**), wie in Anspruch 1 beschrieben.

3. Phytosanitäre Zusammensetzung gemäß Anspruch 2, das eine Suspension des in Anspruch 1 beschriebenen virusbasierten Vektors umfasst.

4. Phytosanitäre Zusammensetzung gemäß einem der Ansprüche 2 oder 3, umfassend in Form eines Sprühmittels zur Inokulation von Pflanzen, nachdem die Mischung in eine an eine Kompressoranlage angeschlossene Airbrush-Pistole eingefüllt wurde, mit der die Pflanzenblätter besprüht werden.

5. **Verfahren** zur Bekämpfung von Anthraknose bei Pflanzen, umfassend die Verabreichung einer ausreichenden Menge einer Phytosanitäre Zusammensetzung gemäß einem der Ansprüche 2 bis 4.

## Revendications

1. **Vecteur à base de virus** (**vecteur Collefree**) destiné à la protection des plantes contre *Colletotrichum acutatum* et/ou *Colletotrichum gloesporioides*, comprenant, dans le génome modifié de l'*Olive mild mosaic virus* (OMMV) ayant le numéro d'accession HQ651834.1, une séquence antimicrobienne provenant d'*Aspergillus niger* ayant le numéro d'accession NT_166523.1, nucléotides 244873 à 245607, ladite séquence étant placée en aval de la CP du génome complet de l'OMMV avec :
- une mutation de l'adénine en thymine au nt 3200 ;
- une mutation de la cytosine en guanine au nt 3359 ;
- une mutation de la guanine en cytosine au nt 3365 ;
- une mutation de la guanine en thymine au nt 3368 ;
- un fragment d'*A. niger* amplifié par les séquences Anafp **SEQ. ID. 1** et **SEQ. ID. 2**, ledit fragment étant introduit au nt 3454.

2. C**omposition phytosanitaire** destinée à la protection des plantes contre *C. acutatum* et/ou *C. gloesporioides*, comprenant un vecteur à base de virus (**vecteur Collefree**) tel que décrit dans la revendication 1.

3. Composition phytosanitaire selon la revendication 2, comprenant une suspension du vecteur à base de virus tel que décrit dans la revendication 1.

4. Composition phytosanitaire selon l'une quelconque des revendications 2 ou 3, se présentant sous la forme d'un spray destiné à inoculer des plantes, après chargement du mélange dans un aérographe relié à un compresseur, ledit mélange étant pulvérisé sur les feuilles des plantes.

5. **Procédé** de lutte contre l'anthracnose des plantes, comprenant l'administration d'une quantité adéquate d'une composition phytosanitaire telle que décrite dans l'une quelconque des revendications 2 à 4.
